(19) Europäisches Patentamt / European Patent Office / Office européen des brevets

(11) **EP 3 719 811 A1**

(12) **EUROPÄISCHE PATENTANMELDUNG**

(43) Veröffentlichungstag:
**07.10.2020  Patentblatt 2020/41**

(51) Int Cl.:
***G16H 50/70*** *(2018.01)*    ***G16H 30/40*** *(2018.01)*

(21) Anmeldenummer: **19166679.1**

(22) Anmeldetag: **02.04.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Siemens Healthcare Diagnostics Inc.
Tarrytown, NY 10591 (US)**

(72) Erfinder:
• **Büttner, Florian
  81541 München (DE)**

• **Geipel, Markus Michael
  80799 München (DE)**
• **Marquardt, Gaby
  91353 Hausen (DE)**
• **Seidel, Daniela
  91083 Baiersdorf (DE)**
• **Tietz, Christoph
  85521 Ottobrunn (DE)**

(74) Vertreter: **Castorph, Simon Johannes
Siemens Healthcare Diagnostics Products GmbH
P.O. Box 22 16 34
80506 München (DE)**

(54) **KONSISTENZ VON DATENKENNZEICHNUNGEN BEI DER MEDIZINISCHEN BILDVERARBEITUNG ZUR ZELLKLASSIFIZIERUNG**

(57)    Die vorliegende Erfindung betrifft ein Computer-implementiertes Verfahren und eine Datenverarbeitungsvorrichtung zur Bereitstellung und Anwendung eines trainierten probabilistischen graphischen Modells bei der medizinischen Bildverarbeitung zur Zellklassifizierung, eine Verwendung des Modells zur Überprüfung und/oder Verbesserung der Konsistenz von Datenkennzeichnungen bei der medizinischen Bildverarbeitung zur Zellklassifizierung, ein Computer-implementiertes Verfahren zur Überprüfung und/oder Verbesserung der Konsistenz von Datenkennzeichnungen bei der medizinischen Bildverarbeitung zur Zellklassifizierung, eine Datenverarbeitungsvorrichtung die ausgebildet ist, die Konsistenz von Datenkennzeichnungen bei der medizinischen Bildverarbeitung zur Zellklassifizierung zu überprüfen und/oder zu verbessern, sowie ein entsprechendes Computerprogrammprodukt und ein computerlesbares Medium.

FIG 2

**Beschreibung**

Die vorliegende Erfindung betrifft ein Computer-implementiertes Verfahren und eine Datenverarbeitungsvorrichtung zur Bereitstellung und Anwendung eines trainierten probabilistischen graphischen Modells bei der medizinischen Bildverarbeitung zur Zellklassifizierung, eine Verwendung des Modells zur Überprüfung und/oder Verbesserung der Konsistenz von Datenkennzeichnungen bei der medizinischen Bildverarbeitung zur Zellklassifizierung, ein Computer-implementiertes Verfahren zur Überprüfung und/oder Verbesserung der Konsistenz von Datenkennzeichnungen bei der medizinischen Bildverarbeitung zur Zellklassifizierung, eine Datenverarbeitungsvorrichtung die ausgebildet ist, die Konsistenz von Datenkennzeichnungen bei der medizinischen Bildverarbeitung zur Zellklassifizierung zu überprüfen und/oder zu verbessern, sowie ein entsprechendes Computerprogrammprodukt und ein computerlesbares Medium.

Die Verfügbarkeit gekennzeichneter Daten ist eine wichtige Voraussetzung für maschinelles Lernen. In der medizinischen Bildverarbeitung werden Datenkennzeichnungen typischerweise von Experten erstellt. Dieser Kennzeichnungsansatz ist aus mehreren Gründen unvorteilhaft: Zunächst ist die Aneignung von Expertenwissen ein sehr zeitaufwändiger und arbeitsintensiver Prozess, der nur nach langjähriger intensiver Praxis befriedigende Resultate liefert. Weiterhin ist das Verfahren fehleranfällig. So können die Expertenbewertungen aufgrund unterschiedlich langer Erfahrung oder aufgrund unterschiedlichen Vorwissens durchaus voneinander abweichen. Insbesondere bei der Bewertung von Zellentwicklungslinien entsprechen die Kennzeichnungen vielfach einer eher künstlichen Kategorisierung mehrerer Stufen einer kontinuierlichen Entwicklung. Hier legen Experten teilweise unterschiedliche Grenzen zwischen Entwicklungsstufen fest oder es kommt zu völligen Fehlklassifizierungen der Zellen.

Wie sich beispielsweise dem Dokument van der Meer et al., 2007, J Clin Pathol, 60(7), 838-839 entnehmen lässt, gibt es in bis zu 10% der Proben Meinungsverschiedenheiten zwischen den bewertenden Experten. Das Dokument erwähnt auch Fälle auffallender Uneinigkeit, wobei in einem Fall einer Zelle fünf verschiedene Bewertungen zugeordnet wurden und über 30% der Experten nicht in der Lage waren, vorhergehende Klassifizierungen zu reproduzieren.

Die von Experten erstellten Klassifikationen sind somit eine relativ unzuverlässige Grundlage für einen Maschinenlern-Ansatz, der auf Mustererkennung ohne Plausibilitätskontrolle basiert.

Der vorliegenden Erfindung liegt somit die Aufgabe zugrunde, ein Verfahren bereitzustellen, das Fehlbewertungen oder falsche Klassifizierungen von Zellen durch Experten vermeidet, Korrekturen von Bewertungen vorschlägt und einem morphologisch analysierenden Fachmann Feedback bezüglich der Plausibilität seiner Einschätzung gibt.

Diese Aufgabe wird durch die Gegenstände der unabhängigen Ansprüche gelöst. In den abhängigen Ansprüchen sind weitere vorteilhafte Aspekte der Erfindung reflektiert.

Die Erfindung betrifft zunächst ein Computer-implementiertes Verfahren zur Erzeugung eines trainierten probabilistischen graphischen Modells bei der medizinischen Bildverarbeitung zur Zellklassifizierung, wobei das Verfahren die folgenden Schritte umfasst:

- Bereitstellen eines Satzes von Trainingsdaten, wobei jeder Trainingsdatensatz Bildinformationen jeweils einer Zelle umfasst;
- Errechnen eines Merkmalsraumes für den Trainingsdatensatz, vorzugsweise mit Hilfe eines Deep Convolutional Neural Network (DCNN);
- Errechnen der Ähnlichkeiten zwischen mindestens zwei Datenpunkten des Trainingsdatensatzes auf Basis des Merkmalsraumes;
- Bereitstellen eines Trainingsdatensatzes in Form von Experten-generierten Datenkennzeichnungen für die entsprechenden Zellen;
- Errechnen von verborgenen Datenkennzeichnungen auf Basis der Ähnlichkeiten zwischen mindestens zwei Datenpunkten des Trainingsdatensatzes und der Experten-generierten Datenkennzeichnungen;
- Erzeugung eines probabilistischen graphischen Modells zur Anpassung der verborgenen Datenkennzeichnungen.

Dieses Verfahren ist auf nicht geordnete Zellen gerichtet. Es bezieht sich somit auf jeden Zelltyp bzw. jede phänotypische Ausprägung von Zellmerkmalen und setzt nicht voraus, dass die zu analysierenden Zellen ähnlich sind, indem sie beispielsweise aus einer inhärenten Entwicklungslinie hervorgegangen sind.

In einem alternativen Ansatz bezieht sich die vorliegende Erfindung auf ein Computer-implementiertes Verfahren zur Erzeugung eines trainierten probabilistischen graphischen Modells bei der medizinischen Bildverarbeitung zur Zellklassifizierung, wobei die Zelle verschiedene Entwicklungsstufen einer Zelle repräsentieren kann, umfassend die folgenden Schritte:

- Bereitstellen eines Satzes von Trainingsdaten, wobei jeder Trainingsdatensatz Bildinformationen einer Zelle umfasst;
- Errechnen eines Merkmalsraumes für den Trainingsdatensatz, vorzugsweise mit Hilfe eines Deep Convolutional Neural Network (DCNN);
- Errechnen der Ähnlichkeiten zwischen mindestens zwei Datenpunkten des Trainingsdatensatzes auf Basis des Merkmalsraumes;
- Errechnen einer Pseudozeit, welche die Datenpunkte gemäß einer Entwicklungsabfolge ordnet;
- Bereitstellen eines Trainingsdatensatzes in Form von Experten-generierten Datenkennzeichnungen für die entsprechenden Zellen;

- Errechnen von verborgenen Datenkennzeichnungen auf Basis der in der Pseudozeit reflektierten Entwicklungsabfolge und der Experten-generierten Datenkennzeichnungen;
- Erzeugung eines probabilistischen graphischen Modells zur Anpassung der verborgenen Datenkennzeichnungen.

Dieses Verfahren ist auf geordnete Zellen gerichtet. Es bezieht sich somit insbesondere auf Zellen oder phänotypische Ausprägungen von Zellmerkmalen, welche ähnlich sind und sich typischerweise aus einer inhärenten Entwicklungslinie ergeben.

Die vorgenannten Computer-implementierten Verfahren werden ergänzt durch Verfahren, welche eine Anpassung der verborgenen Datenkennzeichnungen auf Basis des probabilistischen graphischen Modells vorsehen:

So wird das oben genannte, auf Zellen allgemeiner Art bezogene Verfahren ergänzt durch ein Computer-implementiertes Verfahren, wobei das probabilistische graphische Modell, wie im hier beschriebenen Verfahren erhalten, zur Anpassung von verborgenen Datenkennzeichnungen bei der medizinischen Bildverarbeitung von Zellen verwendet wird, umfassend die Schritte:

- Bereitstellen von verborgenen Datenkennzeichnungen auf Basis der Ähnlichkeiten zwischen mindestens zwei Datenpunkten eines Trainingsdatensatzes und mit diesem Trainingsdatensatz assoziierten Experten-generierten Datenkennzeichnungen, wobei der Trainingsdatensatz Bildinformationen jeweils einer Zelle umfasst;
- Verknüpfen der verborgenen Datenkennzeichnungen und der Experten-generierten Datenkennzeichnungen, wobei die Verknüpfung ein Verbinden aller verborgenen Datenkennzeichnungen untereinander mittels ungerichteter Kanten und ein Verbinden von verborgenen Datenkennzeichnungen und Experten-generierten Datenkennzeichnungen mittels gerichteter Kanten umfasst;
- Anpassen oder Bestätigen der verborgenen Datenkennzeichnungen auf Basis des probabilistischen graphischen Modells

Das oben genannte, auf Zellen einer Entwicklungsreihe bezogene Verfahren wird ergänzt durch ein Computer-implementiertes Verfahren, wobei das probabilistische graphische Modell, wie im hier beschriebenen Verfahren erhalten, zur Anpassung von verborgenen Datenkennzeichnungen bei der medizinischen Bildverarbeitung von Zellen, welche verschiedene Zell-Entwicklungsstufen repräsentieren können, verwendet wird, umfassend die Schritte:

- Bereitstellen von verborgenen Datenkennzeichnungen auf Basis der Ähnlichkeiten zwischen mindestens zwei Datenpunkten eines Trainingsdatensatzes, wobei der Trainingsdatensatz Bildinformationen jeweils einer Zelle umfasst, und einer daraus abgeleiteten Pseudozeit, sowie von mit dem Trainingsdatensatz assoziierten Experten-generierten Datenkennzeichnungen;
- Verknüpfen der verborgenen Datenkennzeichnungen und der Experten-generierten Datenkennzeichnungen, wobei die Verknüpfung ein Verbinden aller Datenkennzeichnungen mittels gerichteter Kanten umfasst;
- Anpassen oder Bestätigen der verborgenen Datenkennzeichnungen auf Basis des probabilistischen graphischen Modells.

Ein Anpassen der verborgenen Datenkennzeichnungen kann beispielsweise eine Korrektur einer bisherigen Zuordnung oder Klassifizierung einer Zelle sein. Dies wäre notwendig, wenn es eine Diskrepanz zwischen der verborgenen Datenkennzeichnung und der Experten-generierten Datenkennzeichnungen gibt. Alternativ kann eine Bestätigung einer bisherigen Zuordnung oder Klassifizierung einer Zelle erfolgen. Dies wäre dann der Fall, wenn es keine Diskrepanz zwischen der verborgenen Datenkennzeichnung und der Experten-generierten Datenkennzeichnungen gibt, also eine identische oder plausible Klassifizierung vorliegt.

Eine Ausführungsform des Errechnens eines Merkmalsraumes für den Trainingsdatensatz, wie in den oben beschriebenen Verfahren erwähnt, sieht die Verwendung eines Maschinenlernalgorithmus für diesen Schritt vor. Unter einem Maschinenlernalgorithmus wird im Kontext dieser Anmeldung insbesondere ein Algorithmus, der zum Maschinellen Lernen ausgebildet ist, verstanden. Maschinenlern-algorithmen werden typischerweise in zwei Klassen unterteilt: Algorithmen des überwachten Lernens("supervised learning") und Algorithmen des nicht-überwachten Lernens ("unsupervised learning").Das überwachte Lernen ist die Fähigkeit von AI-Systemen, Gesetzmäßigkeiten nachzubilden, wobei die Ergebnisse durch Naturbeobachtungen oder Expertenurteile bereits vorhanden sind und genutzt werden, um das System anzulernen. Repräsentative Beispiele für das überwachte Lernen sind der Bayes-Klassifikator, der Naiver Bayes-Klassifikator, die Nächste-Nachbarn-Klassifikation, die Diskriminanzanalyse sowie künstliche Neuronale Netze. Beim überwachten Lernen kann typischerweise eine Funktionenklasse verwendet werden, welche beispielsweise auf Entscheidungsbäumen ("decision trees"), einem Random Forest, einer logistischen Regression, einer Support Vector Machine, Netz, einer Kernel-Methode, oder ähnlichem oder Kombinationen davon basiert. Das unüberwachte Lernen bezeichnet maschinelles Lernen ohne im Voraus bekannte Zielwerte zu kennen. Dabei wird versucht, aus den Eingabedaten Muster zu erkennen. Beispiele sind die automatische Segmentierung (Clustering), sowie die Komprimierung von Daten zur Dimensionsreduktion. Die Komprimierung wird beispielsweise bei der Hauptkomponentenanalyse umgesetzt. Der Maschinenlernalgorithmus kann weiterhin beispielsweise zum tiefen Lernen ("deep learning") und/oder zum bestärkendem Lernen ("reinforcement learning") und/oder zum Marginal Space Learning ausgebildet sein. Mögliche Implementierungen des Maschinenlernalgorithmus können beispielsweise Künstliche Intelligenz verwenden. Für das Optimieren können dem Fachmann bekannte Optimierungsverfahren verwendet werden. Berechnungen, insbesondere beim Optimieren, können beispielsweise mittels eines Prozessorsystems ausgeführt werden. Das Prozessorsystem kann beispielsweise einen oder mehrere Grafikprozessoren aufweisen.

Es ist besonders bevorzugt, dass das Errechnen der Merkmale mit Hilfe eines Deep Convolutional Neural Network (DCNN) durchgeführt wird. Ein Convolutional Neural Network oder faltendes neuronales Netzwerk, ist ein künstliches neuronales Netz, das prinzipiell aus einem oder mehreren Convolutional Layers, gefolgt von einem Pooling Layer besteht. Ein DCNN, wie vorzugsweise im Rahmen der vorliegenden Erfindung verwendet, enthält mehrere Wiederholungen dieser Schichten. Der Convolutional Layer umfasst typischerweise eine zwei- oder dreidimensionale Matrix. Die Aktivität jedes Neurons wird über eine diskrete Faltung berechnet. Ein Neuron in dieser Schicht reagiert nur auf Reize aus seiner lokalen Umgebung. Im Pooling Layer werden im Prinzip überflüssige Informationen verworfen. Eine beispielhafte Umsetzung des Pooling ist das Max-Pooling, bei dem aus jedem 2 x 2 Quadrat aus Neuronen des Convolutional Layers nur die Aktivität des aktivsten Neurons für die weiteren Rechenschritte beibehalten wird. Nach einigen sich wiederholenden Einheiten, bestehend aus Convolutional und Pooling Layer, kann das Netzwerk mit einem oder mehreren Fully-connected Layer abschließen. Die Ausgabe der letzten Schicht des DCNN wird typischerweise durch eine Softmax-Funktion, d.h. einer translationsinvarianten Normalisierung über alle Neuronen im letzten Layer, in eine Wahrscheinlichkeitsverteilung überführt.

Zur Errechnung der Ähnlichkeiten zwischen zwei Datenpunkten aus dem Trainingsdatensatz wird beispielsweise eine Metrik, eine Abstandsfunktion oder ein Kern (Kernel) verwendet. Dabei werden mindestens die beiden Datenpunkte als Eingabewerte verwendet, um als Ausgabewert einen positiven Wert zu erhalten, welcher den Abstand zwischen den Werten beschreibt, wobei die Ähnlichkeit zwischen den Datenpunkten einem reziproken Wert daraus entspricht. In bestimmten Ausführungsformen können zur Errechnung der Ähnlichkeiten nicht nur die Informationen aus den genannten beiden Datenpunkten, sondern ebenfalls Informationen von anderen, vorzugsweise von allen anderen Datenpunkten aus dem Datensatz verwendet werden. Ein Beispiel für einen typischerweise verwendeten Kernel ist

$$K(\mathbf{x}, \mathbf{x}') = \exp\left(-\frac{\|\mathbf{x} - \mathbf{x}'\|^2}{2\sigma^2}\right).$$

Dabei kann zur weiteren Bestimmung der Ähnlichkeiten zwischen zwei Datenpunkten aus dem Trainingsdatensatz auf Basis des Merkmalsraumes, beispielsweis von zwei Bilddaten, vorteilhafterweise ein Ähnlichkeitsgraph erzeugt werden. Dabei werden Ähnlichkeitsgruppen graphisch zugeordnet. Dieses Verfahren ist typischerweise nicht auf Klassifikationsvorwissen angewiesen und somit frei von einer Fehlklassifikationsbeeinflussung.

Trainingsdatensätze für die Verfahren können Bildinformationen jeweils einer Zelle umfassen. Die entsprechenden Informationen können in unterschiedlichen Auflösungen vorliegen. Die Datensätze können ebenfalls qualitative Ergänzungen enthalten. So können die Datensätze Informationen zur Identität der Zellen enthalten. Diese Informationen können den Experten-generierten Datenkennzeichnungen entsprechen. In alternativen Ausführungsformen können die Experten-generierten Datenkennzeichnungen in einem separaten Datensatz enthalten sein. In dieser Konstellation können die Bilddatensätze und die Datenkennzeichnungsdatensätze verlinkt oder mit einander referenziert sein. Die Trainingsdatensätze können darüber hinaus weitere Dateneinheiten umfassen, beispielsweise Informationen zur Aufnahmeeinheit mit welcher sie erzeugt wurden, Informationen zum Zeitpunkt der Erzeugung, optional ebenfalls Patienteninformationen, geographische Informationen oder Informationen zu verwendeten Färbungsverfahren o.ä., sollten diese durchgeführt worden sein.

Die Experten-generierten Datenkennzeichnungen können Informationen zum Zelltyp, zu einer zugrundeliegenden Erkrankung, zum Status der Zellen hinsichtlich der Zellteilungsphase, beispielsweise, ob sich eine Zelle in einer G-, S- oder M-Phase des Zellzyklus befindet, enthalten. Die Datenkennzeichnungen würden in dieser besonderen Ausführungsform nicht eine inhärente Ordnung oder geordneten Zuordnung der Zellen reflektieren.

Für die Verwendung in Verfahren zur Zellklassifizierung von Zellen verschiedener Entwicklungsstufen enthalten diese Datenkennzeichnungen typischerweise Informationen zur Einschätzung bzw. Identifizierung der Entwicklungsstufe der Zellen. Die entsprechenden Informationen können beispielsweise in Form einer Klasseneinteilung oder Entwicklungseinteilung vorliegen und somit eine inhärente Ordnung reflektieren. Die Datenkennzeichnungen können weiterhin Informationen bzgl. einer bereits vorgenommenen Korrektur und/oder einer Bestätigung der Experteneinschätzung enthalten.

Neben den Experten-generierten Datenkennzeichnungen, welche für die Verfahren aus externen Quellen bereitgestellt werden, werden verborgene Datenkennzeichnungen gemäß den beschriebenen Verfahren errechnet. Derartige verborgene Datenkennzeichnungen reflektieren Bewertungen der Zellen hinsichtlich der analysierten Parameter und basieren im Prinzip auf Merkmalsvergleichsoperationen der Trainingsdatensätze mit Bildinformationen zu den Zellen. Die verborgenen Datenkennzeichnungen können mit den entsprechenden, d.h. zu denselben Zellen vorhandenen, Experten-generierten Datenkennzeichnungen assoziiert sein. Hier können ebenfalls Diskrepanzen zwischen den Datenkennzeichnungen registriert und gespeichert werden.

Im Rahmen der Verwendung von Bilddaten zu Zellen verschiedener Entwicklungsstufen, welche einer inhärenten Ordnung folgen, wird eine Pseudozeit im Rahmen der Operationen im Merkmalsraum errechnet. Die Pseudozeit ist ein Konzept, das für Transitionen in biologischen Systemen entwickelt wurde. Hierbei werden individuelle Zellen während der Transition von einem Stadium zum nächsten verfolgt. Da Zellen diese Stadienwechsel typischerweise nicht in einer festgelegten Frequenz durchlaufen, kommt es zu Variationen in der Zeitdimension. Die Pseudozeit wird hierbei als verborgene, d.h. nicht beobachtete Dimension verstanden, welche den Fortschritt der Zellen während der Transitionsvorgänge beschreibt. Weitere Informationen dazu können beispielsweise aus geeigneten Literaturreferenzen wie Reid und Wernisch, 2016, Bioinforamtics, 32(19), 2973-2980 entnommen werden.

Probabilistische graphische Modelle (PGM) wie in den oben genannten Verfahren erhalten bzw. verwendet, sind im Allgemeinen Graphen, deren Knoten Zufallsvariablen sind und in denen die Abwesenheit von Kanten zwischen diesen Knoten deren Unabhängigkeit anzeigt. Die PGM stellen somit einen Formalismus bereit, der es ermöglicht, weitere probabilistische Modelle darzustellen oder umzusetzen.

Eine bevorzugte Ausführungsform des herin beschriebenen Verfahrens, welches auf der Auswertung von Zellen ohne inhärente Ordnung basiert, sieht vor, dass das probabilistische Modell ein Conditional Random Field (CRF) Modell ist. Ein CRF ist ein Typ von ungerichtetem probabilistischem Modell. Typischerweise wird es zur Segmentierung von Sequenzen verwendet. Beispielsweise würde das CRF eine Sequenz x als Eingabe erhalten und eine gleich lange Sequenz y ausgeben. Dabei kann das CRF an jeder Stelle auf die komplette Information der Eingabesequenz zugreifen, was die Verwendung von komplexen Merkmalsmengen erlaubt.

Im Rahmen der Verwendung des CRF wird dabei das Verknüpfen der errechneten Datenkennzeichnung und der Experten-generierten Datenkennzeichnung auf Basis der bedingten Wahrscheinlichkeit der Korrektheit der Experten-generierten Datenkennzeichnung durchgeführt. Dies kann mit verschiedenen Algorithmen umgesetzt werden. Beispielsweise kann auf den Algorithmus Loopy belief propagation, Alpha expansion, Mean field inference oder Linear programming relaxations zurückgegriffen werden.

Eine bevorzugte Ausführungsform des hier beschriebenen Verfahrens, welches auf der Auswertung von Zellen mit inhärenter Ordnung basiert, sieht die Verwendung von Hidden Markov Konzepten vor. Folgen die Zellen beispielsweise einer linearen Entwicklungsabfolge, ist das probabilistische Modell ein Hidden Markov Modell (HMM). Folgen andererseits die Zellen einer dichotomen Entwicklungsabfolge, ist das probabilistische Modell ein Hidden Markov Baum.

Ein HMM ist ein stochastisches Modell, in dem ein System durch eine Markov-Kette mit unbeobachteten Zuständen modelliert wird. Bei der Modellierung als Markov-Kette geht das System typischerweise auf zufällige Weise von einem Zustand in einen anderen über, wobei die Übergangswahrscheinlichkeiten nur jeweils vom aktuellen Zustand abhängen. Hierbei werden diese Zustände nicht extern beobachtet und sind somit verborgen. Jedem dieser inneren Zustände sind dabei beobachtbare Ausgabesymbole (Emissionen) zugeordnet, die je nach Zustand mit gewissen Wahrscheinlichkeiten auftreten. Ein HMM kann als gerichtetes Modell für sequentielle Daten verwendet werden. Dabei greift das HMM nur auf die aktuelle Eingabe zu, nicht jedoch auf die komplette Information der Eingabesequenz.

Ein Hidden Markov Baum ist eine Abwandlung des HMM, wobei die unbeobachteten Zustände in Baumstruktur von einander abhängen bzw. aufeinander folgen. Ein Beispiel, wie ein solcher Algorithmus eingesetzt werden kann, lässt sich dem Dokument Kondo et al., Proceedings of the Eighth Workshop on Statistical Machine Translation, 2013, 503-511, Sofia, Bulgaria entnehmen. Hier wird davon ausgegangen, dass die Ausrichtungsvariablen des Algorithmus eine Baumstruktur haben, die zum Zielabhängigkeitsbaum isomorph ist. Der Algorithmus modelliert die Verzerrungswahrscheinlichkeit basierend auf dem Quell-Abhängigkeitsbaum.

Im Rahmen der Verwendung des HMM oder des Hidden Markov Baumes wird ebenfalls das Verknüpfen der errechneten Datenkennzeichnung und der Experten-generierten Datenkennzeichnung auf Basis der bedingten Wahrscheinlichkeit der Korrektheit der Experten-generierten Datenkennzeichnung durchgeführt. Dabei wird beispielsweise auf den Viterbi Algorithmus zurückgegriffen.

Die Erfindung betrifft ferner eine Datenverarbeitungsvorrichtung zur Erzeugung eines trainierten probabilistischen graphischen Modells bei der medizinischen Bildverarbeitung zur Zellklassifizierung, beispielsweise von ungeordneten Zellen, umfassend:

- eine Einheit zum Bereitstellen eines Satzes von Trainingsdaten, wobei jeder Trainingsdatensatz Bildinformationen einer Zelle umfasst;
- eine Einheit zum Errechnen eines Merkmalsraumes für den Trainingsdatensatz;

- eine Einheit zur Errechnung der Ähnlichkeiten zwischen mindestens zwei Datenpunkten auf Basis des Merkmalsraumes;
- eine Einheit zum Bereitstellen eines Trainingsdatensatz in Form von Experten-generierten Datenkennzeichnungen für die entsprechenden Zellen;
- eine Einheit zur Errechnung von verborgenen Datenkennzeichnungen auf Basis (i) der Ähnlichkeiten zwischen mindestens zwei Datenpunkten und (ii) der Experten-generierten Datenkennzeichnungen;
- eine Einheit zur Erzeugung eines probabilistischen graphischen Modells zur Anpassung der verborgenen Datenkennzeichnungen.

Die Erfindung betrifft ferner eine Datenverarbeitungsvorrichtung zur Erzeugung eines trainierten probabilistischen graphischen Modells bei der medizinischen Bildverarbeitung zur Zellklassifizierung von Zellen, welche beispielsweise verschiedene Entwicklungsstufen einer Zelle repräsentieren und damit einer inhärenten Ordnung unterliegen, umfassend:

- eine Einheit zum Bereitstellen eines Satzes von Trainingsdaten, wobei jeder Trainingsdatensatz Bildinformationen einer Zelle umfasst;
- eine Einheit zum Errechnen eines Merkmalsraumes für den Trainingsdatensatz;
- eine Einheit zur Errechnung der Ähnlichkeiten zwischen mindestens zwei Datenpunkten auf Basis des Merkmalsraumes;
- eine Einheit zur Errechnung einer Pseudozeit, welche die Datenpunkte gemäß einer Entwicklungsabfolge ordnet;
- eine Einheit zum Bereitstellen eines Trainingsdatensatz in Form von Experten-generierten Datenkennzeichnungen für die entsprechenden Zellen;
- eine Einheit zur Errechnung von verborgenen Datenkennzeichnungen auf Basis (i) der in der Pseudozeit reflektierten Entwicklungsabfolge und (ii) der Experten-generierten Datenkennzeichnungen;
- eine Einheit zur Erzeugung eines probabilistischen graphischen Modells zur Anpassung der verborgenen Datenkennzeichnungen.

Weiterhin sieht die Erfindung eine abgewandelte Datenverarbeitungsvorrichtung vor, welche zur Anpassung von verborgenen Datenkennzeichnungen bei der medizinischen Bildverarbeitung von Zellen, beispielsweise von ungeordneten Zellen, auf Basis eines trainierten probabilistisches graphischen Modells verwendet werden kann, wobei die Datenverarbeitungsvorrichtung umfasst:

- eine Einheit zum Bereitstellen von verborgenen Datenkennzeichnungen auf Basis der Ähnlichkeiten zwischen mindestens zwei Datenpunkten eines Trainingsdatensatzes und mit diesem Trainingsdatensatz assoziierten Experten-generierten Datenkennzeichnungen, wobei der Trainingsdatensatz Bildinformationen jeweils einer Zelle umfasst;
- eine Einheit zum Verknüpfen der verborgenen Datenkennzeichnungen und der Experten-generierten Datenkennzeichnungen, wobei die Verknüpfung ein Verbinden aller verborgenen Datenkennzeichnungen untereinander mittels ungerichteter Kanten und ein Verbinden von verborgenen Datenkennzeichnungen und Experten-generierten Datenkennzeichnungen mittels gerichteter Kanten umfasst;
- eine Einheit zum Erhalten von angepassten verborgenen Datenkennzeichnungen auf Basis des probabilistischen graphischen Modells.

Weiterhin sieht die Erfindung eine weitere abgewandelte Datenverarbeitungsvorrichtung vor, welche zur Anpassung von verborgenen Datenkennzeichnungen bei der medizinischen Bildverarbeitung von Zellen, welche beispielsweise verschiedene Entwicklungsstufen einer Zelle repräsentieren und damit einer inhärenten Ordnung unterliegen, auf Basis eines trainierten probabilistisches graphischen Modells verwendet werden kann, wobei die Datenverarbeitungsvorrichtung umfasst:

- eine Einheit zum Bereitstellen von verborgenen Datenkennzeichnungen auf Basis der Ähnlichkeiten zwischen mindestens zwei Datenpunkten eines Trainingsdatensatzes, wobei der Trainingsdatensatz Bildinformationen jeweils einer Zelle umfasst, und einer daraus abgeleiteten Pseudozeit, sowie von mit dem Trainingsdatensatz assoziierten Experten-generierten Datenkennzeichnungen;
- eine Einheit zum Verknüpfen der verborgenen Datenkennzeichnungen und der Experten-generierten Datenkennzeichnungen, wobei die Verknüpfung ein Verbinden aller Datenkennzeichnungen mittels gerichteter Kanten umfasst;
- eine Einheit zum Erhalten von angepassten verborgenen Datenkennzeichnungen auf Basis des probabilistischen graphischen Modells.

Die Erfindung betrifft ferner eine Verwendung eines trainierten probabilistischen graphischen Modells, welches gemäß einem Verfahren zur Erzeugung eines trainierten probabilistischen graphischen Modells nach einem oder mehreren Aspekten dieser Erfindung bereitgestellt wurde, zur Überprüfung und/oder Verbesserung der Konsistenz von Datenkennzeichnungen bei der medizinischen Bildverarbeitung zur Zellklassifizierung.

Die erhaltenen probabilistischen Modelle decken dabei Diskrepanzen zwischen den errechneten verborgenen Datenkennzeichnungen und den Experten-generierten Datenkennzeichnungen auf, welche (i) eine Überprüfung der Experten-generierten Datenkennzeichnungen oder des Analyseverfahrens durch die Experten nahelegt, oder (ii) zu einer Korrektur der Experten-generierten Datenkennzeichnungen führt und somit die Einheitlichkeit der Klassifizierung oder Bewertung von Zellen erhöht.

Insbesondere wird das trainierte probabilistische graphische Modell verwendet, um auf Basis einer abgeleiteten verborgenen Datenkennzeichnung eine Experten-generierte Datenkennzeichnung von Bildinformationen zu korrigieren oder zu bestätigen.

Die Erfindung betrifft ferner ein Computer-implementiertes Verfahren zur Überprüfung und/oder Verbesserung der Konsistenz von Datenkennzeichnungen bei der medizinischen Bildverarbeitung zur Zellklassifizierung, umfassend die Schritte:

- Bereitstellen des Bildes einer Zelle;
- Bereitstellen einer vorhandenen Experten-generierten Datenkennzeichnung;
- Errechnen eines Merkmalsraumes für das Bild;
- Anwenden eines Verfahrens auf Basis eines trainierten probabilistischen graphischen Modells auf die errechneten Merkmale des Bildes;
- Verknüpfen einer Datenkennzeichnung mit dem Bild, wobei das Verknüpfen eine Korrektur oder Bestätigung der bereits vorhandenen Experten-generierten Datenkennzeichnung für dieses Bild umfasst;
- Ausgabe und/oder Speicherung des Bildes zusammen mit der korrigierten oder bestätigten verknüpften Datenkennzeichnung.

In einer Ausführungsform der Erfindung sind die in dem genannten Computer-implementierten Verfahren verwendeten trainierten probabilistischen graphische Modelle jene, welche oben beschrieben sind. Zusätzlich oder alternativ können die anzuwendenden Verfahren jene Verfahren sein, welche oben beschrieben sind.

In einer weiteren Ausführungsform der Erfindung wird das Computer-implementiertes Verfahren wie beschrieben dadurch ergänzt, dass eine Korrektur einer bereits vorhandenen Experten-generierten Datenkennzeichnung zu einer Feedbackanfrage bei einem Experten bezüglich der Datenkennzeichnungsdiskrepanz führt. Damit wird einerseits dem Experten eine Plausibilitätswarnung hinsichtlich seiner Bewertung zugespielt. Gleichzeitig kann die Feedbackgenerierung, ggf. nach einer Neubewertung der Einschätzung durch den Experten oder eine Gruppe von Experten, zu einer Verbesserung des verwendeten Modells oder zur Verbesserung eines abgeleiteten automatisierten Klassifizierungsansatzes führen.

Die Erfindung bezieht sich weiterhin auf eine Datenverarbeitungsvorrichtung, die ausgebildet ist, die Konsistenz von Datenkennzeichnungen bei der medizinischen Bildverarbeitung zur Zellklassifizierung zu überprüfen und/oder zu verbessern, wobei die Vorrichtung mindestens einen Prozessor und einen Speicher aufweist, wobei der mindestens eine Prozessor eingerichtet ist, um einen Programmcode aus dem Speicher zu laden und auszuführen und basierend auf dem Ausführen des Programmcodes die folgenden Schritte auszuführen:

- Bereitstellen des Bildes einer Zelle;
- Bereitstellen einer vorhandenen Experten-generierten Datenkennzeichnung;
- Errechnen eines Merkmalsraumes für das Bild;
- Anwenden eines Verfahrens auf Basis eines trainierten probabilistischen graphischen Modells auf die errechneten Merkmale des Bildes;
- Verknüpfen einer Datenkennzeichnung mit dem Bild, wobei das Verknüpfen eine Korrektur oder Bestätigung der bereits vorhandenen Experten-generierten Datenkennzeichnung für dieses Bild umfasst;
- Ausgabe und/oder Speicherung des Bildes zusammen mit der korrigierten oder bestätigten verknüpften Datenkennzeichnung.

In einer Ausführungsform der Erfindung sind die in der genannten Datenverarbeitungsvorrichtung auszuführenden Schritte dadurch spezifiziert, dass die genannten trainierten probabilistischen graphische Modell jene sind, welche oben beschrieben sind. Zusätzlich oder alternativ können die genannten anzuwendenden Verfahren jene Verfahren sein, welche oben beschrieben sind.

Die Datenverarbeitungsvorrichtung wie hier beschrieben und/oder eine oder mehrere Komponenten davon können von einem Datenverarbeitungssystem gebildet sein. Das Datenverarbeitungssystem kann beispielsweise eine oder mehrere Komponenten in Form von Hardware und/oder eine oder mehrere Komponenten in Form von Software aufweisen.

Das Datenverarbeitungssystem kann beispielsweise zumindest teilweise von einem Cloud-Computing-System gebildet sein. Das Datenverarbeitungssystem kann beispielsweise ein Cloud-Computing-System, ein Computernetzwerk, ein Computer, ein Tablet-Computer, ein Smartphone oder ähnliches oder eine Kombination davon sein und/oder aufweisen.

Die Hardware kann beispielsweise mit einer Software zusammenwirken und/oder mittels einer Software konfigurierbar sein. Die Software kann beispielsweise mittels der Hardware ausgeführt werden. Bei der Hardware kann es sich beispielsweise um ein Speichersystem, ein FPGA-System (Fieldprogrammable gate array), ein ASIC-System (Applicationspecific integrated circuit), ein Mikrocontroller-System, ein Prozessorsystem und Kombinationen davon handeln. Das Prozessorsystem kann beispielsweise einen Mikroprozessor und/oder mehrere zusammenwirkende Mikroprozessoren aufweisen. Insbesondere kann eine Komponente der Datenverarbeitungsvorrichtung nach einem der Aspekte, die in dieser Anmeldung offenbart sind, welche dazu ausgebildet ist, einen gegebenen Schritt eines Verfahrens nach einem der Aspekte, die in dieser Anmeldung offenbart sind, auszuführen, in Form einer Hardware implementiert sein, welche zum Ausführen des gegebenen Schritts konfiguriert ist und/oder welche zum Ausführen einer computerlesbaren Anweisung derart konfiguriert ist, dass die Hardware mittels der computerlesbaren Anweisung zum Ausführen des gegebenen Schritts konfigurierbar ist. Insbesondere kann das System einen Speicherbereich, beispielsweise in Form eines computerlesbaren Mediums, aufweisen, in welchem computerlesbare Anweisungen, beispielsweise in Form eines Computerprogramms, gespeichert sind.

Ein Datentransfer zwischen Komponenten des Datenverarbeitungssystems kann beispielsweise jeweils mittels einer geeigneten Datentransfer-Schnittstelle erfolgen. Die Datentransfer-Schnittstelle zum Datentransfer an und/oder von einer Komponente des Datenverarbeitungssystems kann zumindest teilweise in Form von Software und/oder zumindest teilweise in Form von Hardware realisiert sein. Die Datentransfer-Schnittstelle kann beispielsweise zum Abspeichern von Daten in und/oder zum Laden von Daten aus einem Bereich des Speichersystems ausgebildet sein, wobei auf diesen Bereich des Speichersystems eine oder mehrere Komponenten des Datenverarbeitungssystems zugreifen können.

Die Erfindung bezieht sich weiterhin auf ein Computerprogrammprodukt mit einem Computerprogramm, welches direkt in eine Speichereinrichtung eines Computers ladbar ist, mit Programmabschnitten, um alle Schritte eines Verfahrens nach einem oder mehreren der hierin genannten Aspekte auszuführen, wenn das Computerprogramm in dem Computer ausgeführt wird.

Das Computerprogramm ist in das Speichersystem des Datenverarbeitungssystems ladbar und von dem Prozessorsystem des Datenverarbeitungssystems ausführbar. Das Datenverarbeitungssystem kann beispielsweise mittels des Computerprogramms derart ausgebildet sein, dass das Datenverarbeitungssystem die Schritte eines Verfahrens nach einer der Ausführungsformen, die in dieser Anmeldung genannte sind, ausführen kann, wenn das Computerprogramm von dem Datenverarbeitungssystem ausgeführt wird.

Das Computerprogrammprodukt kann beispielsweise das Computerprogramm sein oder neben dem Computerprogramm mindestens einen zusätzlichen Bestandteil umfassen. Der mindestens eine zusätzliche Bestandteil des Computerprogrammprodukts kann als Hardware und/oder als Software ausgebildet sein. Das Computerprogrammprodukt kann beispielsweise ein Speichermedium, auf dem zumindest ein Teil des Computerprogrammprodukts gespeichert ist, und/oder ein Schlüssel zur Authentifizierung eines Benutzers des Computerprogrammprodukts, insbesondere in Form eines Dongles, aufweisen.

Das Computerprogrammprodukt und/oder das Computerprogramm kann beispielsweise ein Cloud-Anwendungs-Programm aufweisen, welches zum Verteilen von Programmabschnitten des Computerprogramms auf verschiedene Verarbeitungseinheiten, insbesondere verschiedene Computer, eines Cloud-Computing-Systems ausgebildet ist, wobei jede der Verarbeitungseinheiten zum Ausführen eines oder mehrerer Programmabschnitte des Computerprogramms ausgebildet ist.

Die Erfindung bezieht sich weiterhin auf ein computerlesbares Medium, auf welchem von einem Computer einlesbare und ausführbare Programmabschnitte gespeichert sind, um alle Schritte eines Verfahrens nach einem oder mehreren der hierin genannten Aspekte auszuführen, wenn die Programmabschnitte von dem Computer ausgeführt werden.

Auf dem computerlesbaren Medium kann beispielsweise das Computerprogrammprodukt nach einer der Ausführungsformen, die in dieser Anmeldung offenbart sind, und/oder das Computerprogramm nach einer der Ausführungsformen, die in dieser Anmeldung offenbart sind, gespeichert sein.

Das computerlesbare Medium kann beispielsweise ein Memorystick, eine Festplatte oder ein sonstiger Datenträger sein, der insbesondere lösbar mit dem Datenverarbeitungssystem verbunden oder fest in das Datenverarbeitungssystem integriert sein kann. Das computerlesbare Medium kann beispielsweise einen Bereich des Speichersystems des Datenverarbeitungssystems bilden.

Die erfindungsgemäße Lösung im Kontext der oben beschriebenen Aspekte ermöglicht somit die Verbesserung eines ebenfalls hierin enthaltenen allgemeinen Klassifizierungsverfahrens zur Zellzuordnung oder Zelleinteilung, welches auf einem simplen Maschinenlernalgorithmus ohne Plausibilitätskontrolle basiert. Dabei wird vor allem die Datenqualität erhöht, sodass eine genauere und damit auch kosten-effizientere automatische Klassifizierung umsetzbar wird. Darüber hinaus ermöglichen einige der hierin genannten erfindungsgemäßen Gegenstände die Festlegung von konsistenteren und reproduzierbareren Grenzen zwischen Zellentwicklungsstufen. Dabei werden die Experten-generierten Datenkennzeichnungen konsolidiert und somit breiter einsetzbar. Weiterhin kann die Feedback-Generierung, wie oben ausgeführt, zur Erstellung neuer Richtlinien oder Regeln zur manuellen Bewertung beitragen. Darüber hinaus kann die erfindungsgemäße Lösung auch zu einer Verbesserung des Dialogs zwischen dem Experten und einem Maschinenlern-Ingenieur führen, was zu einer Verbesserung der Datenkonsistenz beiträgt.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, soweit er durch die Ansprüche vorgegeben ist.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Ausdrucks "aufweisen" verknüpften Begriffe identisch sein können. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander getrennt sind. Der Ausdruck "basierend auf" oder "auf Basis von" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

Im Folgenden wird die Erfindung anhand von weiteren Ausführungsbeispielen unter Hinweis auf die beigefügten Figuren erläutert. Die Darstellung in den Figuren ist schematisch, stark vereinfacht und nicht zwingend maßstabsgetreu.

Es zeigen:

**Figur 1** den Status Quo des maschinellen Lernprozesses aus dem Stand der Technik.

**Figur 2** ein schematisches Berechnungsverfahren auf Basis ungeordneter Zellen.

**Figur 3** die Verknüpfung von Datenkennzeichnungen für ungeordnete Zellen.

**Figur 4** die Entwicklungsstadien eines Granulozyten. Von links nach rechts sind die Stadien Myeloblast, Promyelozyt, Myelozyt, Metamyelozy und stabkerniger neutrophiler segmentkerniger Granulozyt dargestellt.

**Figur 5** ein schematisches Berechnungsverfahren für Zellen, welche einer Transition von Zellentwicklungsstadien unterliegen.

**Figur 6** ein Deep Convolutional Neural Network für die Berechnung eines Merkmalsraumes für Trainingsdatensätze.

**Figur 7** die Darstellung von Zellen im Merkmalsraum. Die abgeleiteten Informationen zu den Zellen sind in Form eines Ähnlichkeitsgraphen angeordnet, wobei der Kurvenverlauf im Verhältnis zum Entwicklungsstadium der Zellen verläuft.

**Figur 8** die Reihenfolge von Zellen in der Pseudozeit, wobei eine Verknüpfung zwischen der Pseudozeit und der Repräsentation gem. Bilddaten hergestellt werden kann.

**Figur 9** die Verknüpfung von verborgenen Datenkennzeichnungen und Experten-generierten Datenkennzeichnungen als Ausgangssituation für die Anwendung eines Hidden Markov Models.

**Figur 10** die Verknüpfung von verborgenen Datenkennzeichnungen und Experten-generierten Datenkennzeichnungen nach Anwendung eines Hidden Markov Models.

**Figur 11** Diskrepanzen zwischen den verborgenen Datenkennzeichnungen und den Experten-generierten Datenkennzeichnungen welche zur Aufdeckung von Fehlbewertungen führen können.

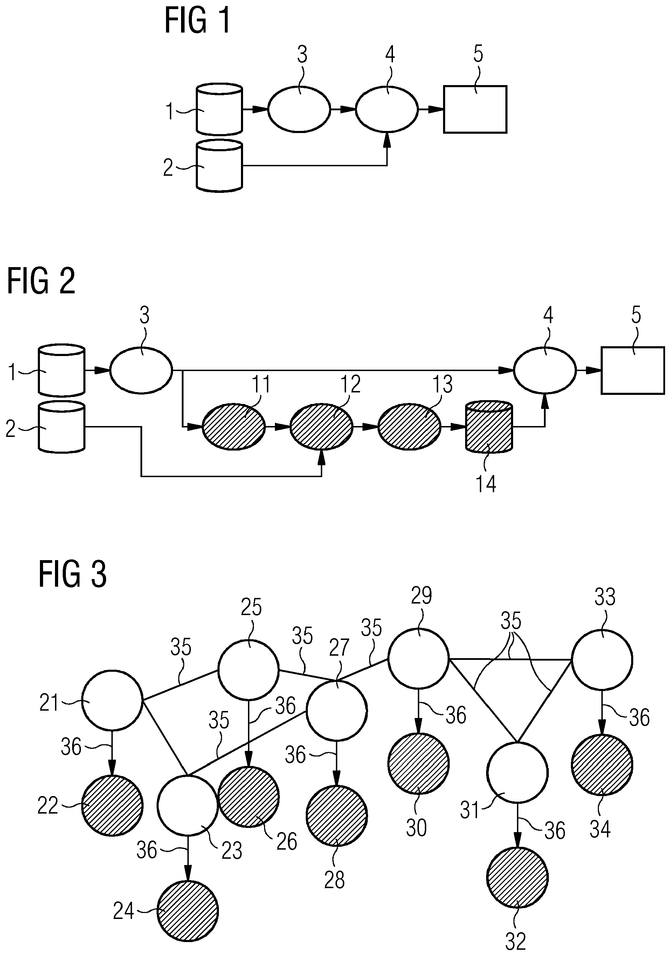

**Figur 1** zeigt den Status Quo des maschinellen Lernprozesses aus dem Stand der Technik. Dabei wird mit Bilddaten **1** eine Merkmalsberechnung **3** durchgeführt. Die Ergebnisse werden einem Modul für Maschinenlernen **4** zugeführt. Diesem Modul werden ebenfalls Experten-generierte Datenkennzeichnungen **2** zugeführt. Nach Durchlaufen eines Maschinenlernprozesses **4** kann ein Klassifizierungsmodell **5** erstellt werden.

**Figur 2** zeigt ein erfindungsgemäßes schematisches Berechnungsverfahren auf Basis ungeordneter Zellen bzw. willkürlicher Klassen. Dabei wird mit Bilddaten **1** zunächst eine Merkmalsberechnung **3** durchgeführt. Während die bisherigen Verfahren die Ergebnisse dieser Berechnung einem Modul für Maschinenlernen **4** direkt zugeführt haben, basiert die Erfindung darauf, ein Datenkennzeichnungskorrektur-Subsystem **(11, 12, 13** und **14)** zu etablieren. Dabei wird zunächst ein Ähnlichkeitsgraph **11** errechnet. In einem nächsten Schritt wird ein probabilistisches Modell in Form eines Conditional Random Field (CRF) **12** erstellt. Dieses Modell speist sich ebenfalls aus Experten-generierten Datenkennzeichnungen **2**.

Anschließend können nach Anwendung des CRF verborgene Datenkennzeichnungen **13** abgeleitet werden. Dies führt zu einer möglichen Korrektur der verborgenen Datenkennzeichnungen **14**. Diese werden dann einem Modul für Maschinenlernen **4** zugeführt, das schließlich ein Klassifizierungsmodell **5** generieren kann.

**Figur 3** zeigt schematisch die Verknüpfung der Datenkennzeichnungen für ungeordnete Zellen. Hier wird eine verborgene Datenkennzeichnung von Zelle A **21** mit einer Experten-generierte Datenkennzeichnung von Zelle A **22,** eine verborgene Datenkennzeichnung von Zelle B **23** mit einer Experten-generierte Datenkennzeichnung von Zelle B **24,** eine verborgene Datenkennzeichnung von Zelle C **25** mit einer Experten-generierte Datenkennzeichnung von Zelle C **26,** eine verborgene Datenkennzeichnung von Zelle D **27** mit einer Experten-generierte Datenkennzeichnung von Zelle D **28,** eine verborgene Datenkennzeichnung von Zelle E **29** mit einer Experten-generierte Datenkennzeichnung von Zelle E **30,** eine verborgene Datenkennzeichnung von Zelle F **31** mit einer Experten-generierte Datenkennzeichnung von Zelle F **32** und eine verborgene Datenkennzeichnung von Zelle G **33** mit einer Experten-generierte Datenkennzeichnung von Zelle G **34** mittels gerichteter Kanten verknüpft **36.** Eine Verknüpfung der verborgenen Datenkennzeichnungen untereinander ist mittels ungerichteter Kanten **35** vorgesehen.

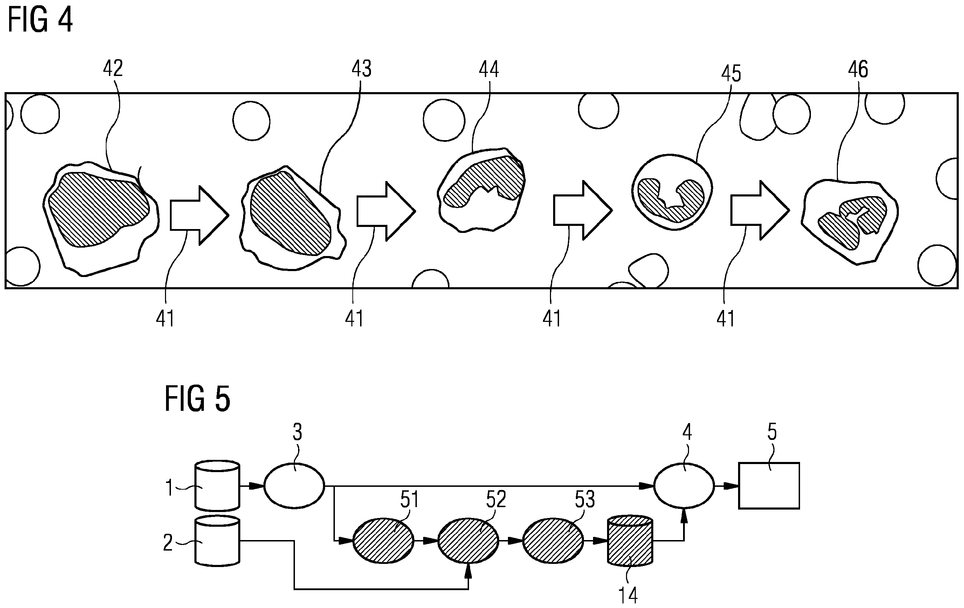

**Figur 4** zeigt eine Transition zwischen verschiedenen Zellentwicklungsstadien **41** am Beispiel von Granulozyten. Von links nach rechts sind die Stadien Myeloblast **42,** Promyelozyt **43,** Myelozyt **44,** Metamyelozyt **45** und stabkerniger neutrophiler segmentkerniger Granulozyt **46** dargestellt.

Die abgebildeten Zellen entsprechen einer geordneten Abfolge und unterscheiden sich von willkürlichen Klassen durch inhärente Ordnungsprinzipien, wie ähnlichen Aufbau, ähnliche Größe, aber Unterschieden in der Morphologie des Zellkerns etc.

**Figur 5** zeigt ein erfindungsgemäßes schematisches Berechnungsverfahren auf Basis von Zellen, welche einer Transition von Zellentwicklungsstadien unterliegen. Dabei wird mit Bilddaten **1** zunächst eine Merkmalsberechnung **3** durchgeführt. Während die bisherigen Verfahren die Ergebnisse dieser Berechnung einem Modul für Maschinenlernen **4** direkt zugeführt haben, basiert die Erfindung darauf, ein Datenkennzeichnungskorrektur-Subsystem **(51, 52, 53** und **14)** zu etablieren. Dabei wird zunächst eine Pseudozeit **51** errechnet. In einem nächsten Schritt wir ein probabilistisches Modell in Form eines Hidden Markov Modells (HMM) **52** erstellt. Dieses Modell speist sich ebenfalls aus Experten-generierten Datenkennzeichnungen **2.**

Anschließend können nach Anwendung des HMM Datenkennzeichnungsgrenzen in der Pseudozeit **53** abgeleitet werden. Dies führt zu einer möglichen Korrektur der verborgenen Datenkennzeichnungen **14.** Diese werden dann einem Modul für Maschinenlernen **4** zugeführt, das schließlich ein Klassifizierungsmodell **5** generieren kann.

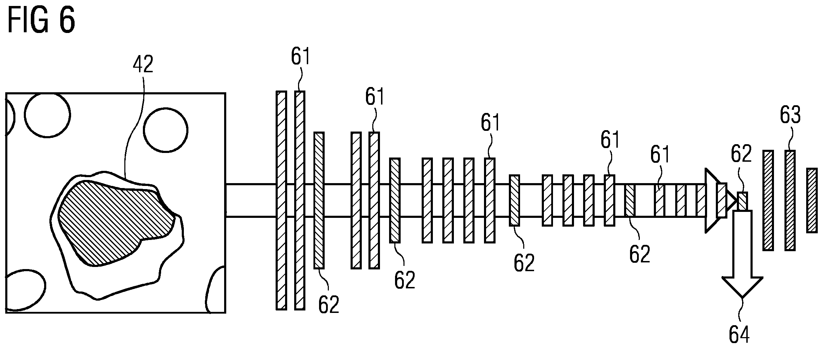

**Figur 6** zeigt ein Deep Convolutional Neural Network (DCNN) für die Berechnung eines Merkmalsraumes für Trainingsdatensätze. Ausgangspunkt ist das Bild eines Myeloblasten **42** als Trainingsdatensatz, dessen Merkmale mittels eines Neuronalen Netzwerkes durch Faltung **61,** Max-Pooling **62** und Verdichtung **63** analysiert werden, um eine Repräsentation im Merkmalsraum **64** zu erhalten.

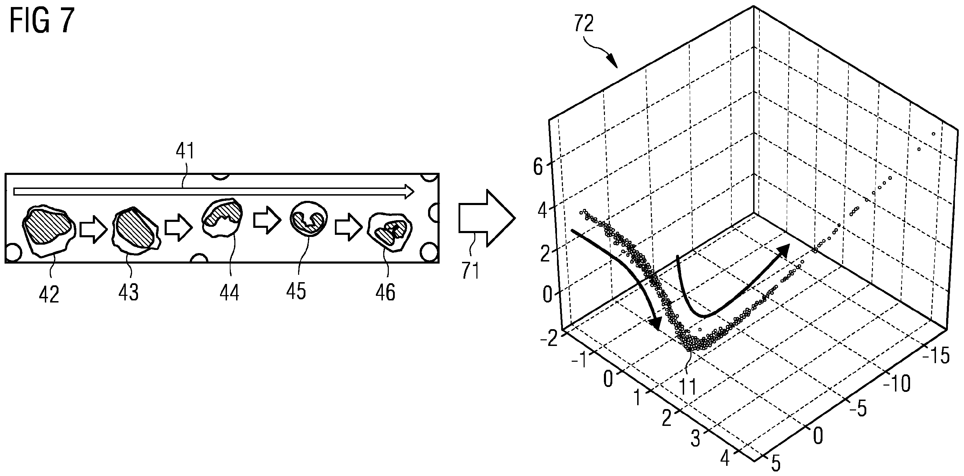

**Figur 7** zeigt die Darstellung von Zellen im Merkmalsraum. Im linken Bildbereich ist die Transition zwischen verschiedenen Zellentwicklungsstadien **41** am Beispiel von Granulozyten zu erkennen. Von links nach rechts sind die Stadien Myeloblast **42,** Promyelozyt **43,** Myelozyt **44,** Metamyelozyt **45** und stabkerniger neutrophiler segmentkerniger Granulozyt **46** dargestellt. Nach einer Transformation **71** wird ein Merkmalsraum **72** erstellt, welcher in Form eines Ähnlichkeitsgraphen **11** repräsentiert sein kann. Die abgeleiteten Informationen zu den Zellen sind dabei in Form des Ähnlichkeitsgraphen **11** angeordnet, wobei der Kurvenverlauf im Verhältnis zum Entwicklungsstadium der Zellen angeordnet ist.

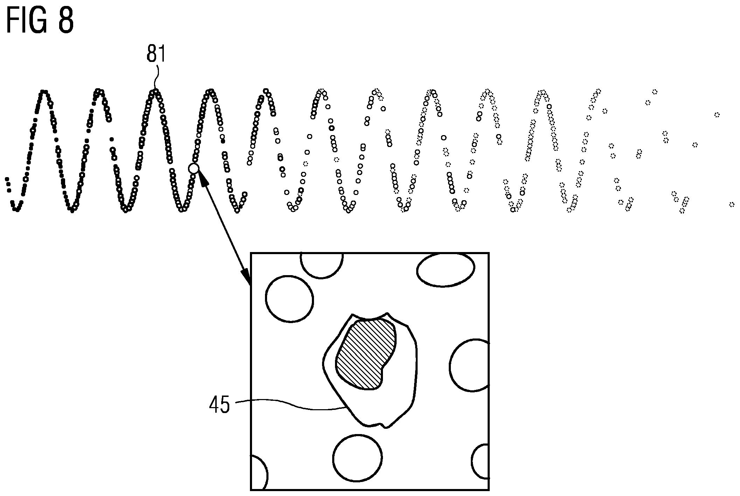

**Figur 8** reflektiert die Reihenfolge von Zellen in der Pseudozeit **81,** wobei eine Verknüpfung zwischen der Pseudozeit und der Repräsentation gem. Bilddaten hergestellt werden kann. Als Beispiel ist die Positionierung eines Metamyelozyten **45** dargestellt.

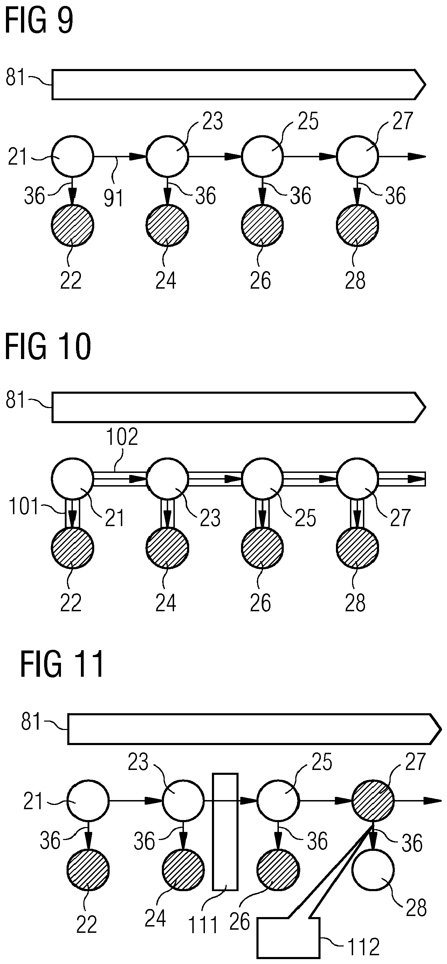

**Figur 9** zeigt die Verknüpfung von verborgenen Datenkennzeichnungen und Experten-generierten Datenkennzeichnungen als Ausgangssituation für die Anwendung eines Hidden Markov Models im Rahmen der Analyse von Zellen die einer Transition zwischen verschiedenen Zellentwicklungsstadien unterliegen, also nicht ungeordnet sind. Diese werden zunächst im Rahmen der errechneten Pseudozeit **81** angeordnet. Anschließend werden die verborgene Datenkennzeichnung von Zelle A **21** mit einer Experten-generierte Datenkennzeichnung von Zelle A **22,** eine verborgene Datenkennzeichnung von Zelle B **23** mit einer Experten-generierte Datenkennzeichnung von Zelle B **24,** eine verborgene Datenkennzeichnung von Zelle C **25** mit einer Experten-generierte Datenkennzeichnung von Zelle C **26,** eine verborgene Datenkennzeichnung von Zelle D **27** mit einer Experten-generierte Datenkennzeichnung von Zelle D **28** verknüpft. Der Abstand zwischen den Zellen im Rahmen des Pseudozeitkonzeptes wird als Distanz zur nächsten Zelle in der Pseudozeit **91** gezeigt. Die verborgenen Datenkennzeichnungen und die Experten-generierten Datenkennzeichnungen sind mittels gerichteter Kanten **36** verknüpft.

**Figur 10** zeigt die Verknüpfung von verborgenen Datenkennzeichnungen und Experten-generierten Datenkennzeichnungen nach Anwendung eines Hidden Markov Models im Rahmen der Analyse von Zellen die einer Transition zwischen verschiedenen Zellentwicklungsstadien unterliegen, also nicht ungeordnet sind. Diese werden zunächst im Rahmen der errechneten Pseudozeit **81** angeordnet. Anschließend werden die verborgene Datenkennzeichnung von Zelle A **21** mit einer Experten-generierte Datenkennzeichnung von Zelle A **22,** eine verborgene Datenkennzeichnung von Zelle B **23** mit einer Experten-generierte Datenkennzeichnung von Zelle B **24,** eine verborgene Datenkennzeichnung von Zelle C **25** mit einer Experten-generierte Datenkennzeichnung von Zelle C **26,** eine verborgene Datenkennzeichnung von Zelle D **27** mit einer Experten-generierte Datenkennzeichnung von Zelle D **28** verknüpft. Hierbei erfolgt eine Codierung der Verknüpfungen durch die Transitionsmatrix des HMM **102,** sowie eine Codierung in Emissionswahrscheinlichkeit des HMM **101.** Die verborgenen Datenkennzeichnungen und die Experten-generierten Datenkennzeichnungen sind mittels gerichteter Kanten **36** verknüpft.

**Figur 11** zeigt Diskrepanzen zwischen den verborgenen Datenkennzeichnungen und den Experten-generierten Datenkennzeichnungen welche zur Aufdeckung von Fehlbewertungen bzw. Fehlklassifikationen führen können. Zunächst erfolgte eine Anordnung im Rahmen der errechneten Pseudozeit **81**. Nach einer Verknüpfung der verborgene Datenkennzeichnung von Zelle A **21** mit einer Experten-generierte Datenkennzeichnung von Zelle A **22,** der verborgene Datenkennzeichnung von Zelle B **23** mit einer Experten-generierte Datenkennzeichnung von Zelle B **24,** der verborgenen Datenkennzeichnung von Zelle C **25** mit einer Experten-generierte Datenkennzeichnung von Zelle C **26,** und der verborgene Datenkennzeichnung von Zelle D **27** mit einer Experten-generierte Datenkennzeichnung von Zelle D **28** kann zunächst eine geschätzte Klassen- bzw. Entwicklungsstadiengrenze **111** etabliert werden. Die verborgenen Datenkennzeichnungen und die Experten-generierten Datenkennzeichnungen sind dabei mittels gerichteter Kanten **36** verknüpft. Hier zeigt sich jedoch, dass eine Experten-generierte Datenkennzeichnung **28** auf eine andere Klasse verweist, als die damit assoziierte, in der Pseudozeit jüngere verborgene Datenkennzeichnung **27**. Dies deutet auf einen Bewertungsfehler des Experten **112** hin.

Die identifizierte Diskrepanz mit vermutetem Bewertungsfehler zwischen den Datenkennzeichnungen kann anschließend in Form eines Feedbacks an den Experten zurückgespielt werden, sodass eine Neubewertung oder Überprüfung möglich ist. Damit ist unter anderem ein effizientes Assistenzsystem zur Zellklassifizierung umsetzbar.

Bezugszeichen

1 Bilddaten
2 Experten-generierte Datenkennzeichnungen
3 Merkmalsberechnung
4 Maschinenlernen
5 Klassifizierungsmodell
11 Berechnung des Ähnlichkeitsgraph
12 Erstellung eines probabilistischen Modells in Form eines Conditional Random Field (CRF)
13 Ableitung von verborgenen Datenkennzeichnungen
14 Korrektur der verborgenen Datenkennzeichnungen
21 Verborgene Datenkennzeichnung von Zelle A
22 Experten-generierte Datenkennzeichnung von Zelle A
23 Verborgene Datenkennzeichnung von Zelle B
24 Experten-generierte Datenkennzeichnung von Zelle B
25 Verborgene Datenkennzeichnung von Zelle C
26 Experten-generierte Datenkennzeichnung von Zelle C
27 Verborgene Datenkennzeichnung von Zelle D
28 Experten-generierte Datenkennzeichnung von Zelle D
29 Verborgene Datenkennzeichnung von Zelle E
30 Experten-generierte Datenkennzeichnung von Zelle E
31 Verborgene Datenkennzeichnung von Zelle F
32 Experten-generierte Datenkennzeichnung von Zelle F
33 Verborgene Datenkennzeichnung von Zelle G
34 Experten-generierte Datenkennzeichnung von Zelle G
35 Verknüpfung der verborgenen Datenkennzeichen untereinander
36 Verknüpfung der verborgenen Datenkennzeichen und der Experten-generierte Datenkennzeichnungen
41 Transition von Zellentwicklungsstadien
42 Myeloblast
43 Promyelozyt
44 Myelozyt
45 Metamyelozyt
46 stabkerniger neutrophiler segmentkerniger Granulozyt
51 Berechnung der Pseudozeit
52 Erstellung eines probabilistischen Modells in Form eines Hidden Markov Modells (HMM)
53 Ableitung von der Datenkennzeichnungsgrenzen in der Pseudozeit
61 Faltung
62 Max-Pooling
63 Verdichtung
64 Repräsentation im Merkmalsraum
71 Transformation
72 Merkmalsraum
73 Zellen im Rahmen ihrer Entwicklung im Merkmalsraum
81 Zellen in der Pseudozeit
91 Distanz zur nächsten Zelle in der Pseudozeit
101 Codierung in Emissionswahrscheinlichkeit des HMM
102 Codierung durch Transitionsmatrix des HMM
111 geschätzte Klassen- bzw. Entwicklungsstadiengrenze
112 Fehler des Experten

**Patentansprüche**

1. Computer-implementiertes Verfahren zur Erzeugung eines trainierten probabilistischen graphischen Modells bei der medizinischen Bildverarbeitung zur Zellklassifizierung, wobei das Verfahren die folgenden Schritte umfasst:

    - Bereitstellen eines Satzes von Trainingsdaten, wobei jeder Trainingsdatensatz Bildinformationen jeweils einer Zelle umfasst;

- Errechnen eines Merkmalsraumes für den Trainingsdatensatz, vorzugsweise mit Hilfe eines Deep Convolutional Neural Network (DCNN);
- Errechnen der Ähnlichkeiten zwischen mindestens zwei Datenpunkten des Trainingsdatensatzes auf Basis des Merkmalsraumes;
- Bereitstellen eines Trainingsdatensatzes in Form von Experten-generierten Datenkennzeichnungen für die entsprechenden Zellen;
- Errechnen von verborgenen Datenkennzeichnungen auf Basis der Ähnlichkeiten zwischen mindestens zwei Datenpunkten des Trainingsdatensatzes und der Experten-generierten Datenkennzeichnungen;
- Erzeugung eines probabilistischen graphischen Modells zur Anpassung der verborgenen Datenkennzeichnungen, wobei das probabilistische Modell vorzugsweise ein Conditional Random Field (CRF) Modell ist.

2.  Computer-implementiertes Verfahren, wobei das probabilistische graphische Modell, wie in Anspruch 1 erhalten, zur Anpassung von verborgenen Datenkennzeichnungen bei der medizinischen Bildverarbeitung von Zellen verwendet wird, umfassend die Schritte:

- Bereitstellen von verborgenen Datenkennzeichnungen auf Basis der Ähnlichkeiten zwischen mindestens zwei Datenpunkten eines Trainingsdatensatzes und mit diesem Trainingsdatensatz assoziierten Experten-generierten Datenkennzeichnungen, wobei der Trainingsdatensatz Bildinformationen jeweils einer Zelle umfasst;
- Verknüpfen der verborgenen Datenkennzeichnungen und der Experten-generierten Datenkennzeichnungen, wobei die Verknüpfung ein Verbinden aller verborgenen Datenkennzeichnungen untereinander mittels ungerichteter Kanten und ein Verbinden von verborgenen Datenkennzeichnungen und Experten-generierten Datenkennzeichnungen mittels gerichteter Kanten umfasst;
- Anpassen oder Bestätigen der verborgenen Datenkennzeichnungen auf Basis des probabilistischen graphischen Modells.

3.  Computer-implementiertes Verfahren zur Erzeugung eines trainierten probabilistischen graphischen Modells bei der medizinischen Bildverarbeitung zur Zellklassifizierung, wobei die Zelle verschiedene Entwicklungsstufen einer Zelle repräsentieren kann, umfassend die folgenden Schritte:

- Bereitstellen eines Satzes von Trainingsdaten, wobei jeder Trainingsdatensatz Bildinformationen einer Zelle umfasst;
- Errechnen eines Merkmalsraumes für den Trainingsdatensatz, vorzugsweise mit Hilfe eines Deep Convolutional Neural Network (DCNN);
- Errechnen der Ähnlichkeiten zwischen mindestens zwei Datenpunkten des Trainingsdatensatzes auf Basis des Merkmalsraumes;
- Errechnen einer Pseudozeit, welche die Datenpunkte gemäß einer Entwicklungsabfolge ordnet;
- Bereitstellen eines Trainingsdatensatzes in Form von Experten-generierten Datenkennzeichnungen für die entsprechenden Zellen;
- Errechnen von verborgenen Datenkennzeichnungen auf Basis der in der Pseudozeit reflektierten Entwicklungsabfolge und der Experten-generierten Datenkennzeichnungen;
- Erzeugung eines probabilistischen graphischen Modells zur Anpassung der verborgenen Datenkennzeichnungen, wobei vorzugsweise das probabilistische Modell bei einer linearen Entwicklungsabfolge ein Hidden Markov Modell (HMM) und bei einer dichotomen Entwicklungsabfolge ein Hidden Markov Baum ist.

4.  Computer-implementiertes Verfahren, wobei das probabilistische graphische Modell, wie in Anspruch 3 erhalten, zur Anpassung von verborgenen Datenkennzeichnungen bei der medizinischen Bildverarbeitung von Zellen, welche verschiedene Zell-Entwicklungsstufen repräsentieren können, verwendet wird, umfassend die Schritte:

- Bereitstellen von verborgenen Datenkennzeichnungen auf Basis der Ähnlichkeiten zwischen mindestens zwei Datenpunkten eines Trainingsdatensatzes, wobei der Trainingsdatensatz Bildinformationen jeweils einer Zelle umfasst, und einer daraus abgeleiteten Pseudozeit, sowie von mit dem Trainingsdatensatz assoziierten Experten-generierten Datenkennzeichnungen;
- Verknüpfen der verborgenen Datenkennzeichnungen und der Experten-generierten Datenkennzeichnungen, wobei die Verknüpfung ein Verbinden aller Datenkennzeichnungen mittels gerichteter Kanten umfasst;
- Anpassen oder Bestätigen der verborgenen Datenkennzeichnungen auf Basis des probabilistischen graphischen Modells.

5.  Computer-implementiertes Verfahren nach einem der Ansprüche 1 oder 3, wobei die Errechnung der Ähnlichkeiten zwischen mindestens zwei Datenpunkten die Erstellung eines Ähnlichkeitsgraphen umfasst.

**6.** Computer-implementiertes Verfahren nach einem der Ansprüche 2 oder 4, wobei das Verknüpfen der errechneten Datenkennzeichnung und der Experten-generierten Datenkennzeichnung auf Basis der bedingten Wahrscheinlichkeit der Korrektheit der Experten-generierten Datenkennzeichnung durchgeführt wird.

**7.** Datenverarbeitungsvorrichtung zur Erzeugung eines trainierten probabilistischen graphischen Modells bei der medizinischen Bildverarbeitung zur Zellklassifizierung, umfassend:

- eine Einheit zum Bereitstellen eines Satzes von Trainingsdaten, wobei jeder Trainingsdatensatz Bildinformationen einer Zelle umfasst;
- eine Einheit zum Errechnen eines Merkmalsraumes für den Trainingsdatensatz;
- eine Einheit zur Errechnung der Ähnlichkeiten zwischen mindestens zwei Datenpunkten auf Basis des Merkmalsraumes;
- optional eine Einheit zur Errechnung einer Pseudozeit, welche die Datenpunkte gemäß einer Entwicklungsabfolge ordnet;
- eine Einheit zum Bereitstellen eines Trainingsdatensatz in Form von Experten-generierten Datenkennzeichnungen für die entsprechenden Zellen;
- eine Einheit zur Errechnung von verborgenen Datenkennzeichnungen auf Basis (i) der Ähnlichkeiten zwischen mindestens zwei Datenpunkten oder auf Basis der in der Pseudozeit reflektierten Entwicklungsabfolge und (ii) der Experten-generierten Datenkennzeichnungen;
- eine Einheit zur Erzeugung eines probabilistischen graphischen Modells zur Anpassung der verborgenen Datenkennzeichnungen.

**8.** Datenverarbeitungsvorrichtung zur Anpassung von verborgenen Datenkennzeichnungen bei der medizinischen Bildverarbeitung von Zellen auf Basis eines trainierten probabilistisches graphischen Modells, umfassend:

- eine Einheit zum (i) Bereitstellen von verborgenen Datenkennzeichnungen auf Basis der Ähnlichkeiten zwischen mindestens zwei Datenpunkten eines Trainingsdatensatzes und mit diesem Trainingsdatensatz assoziierten Experten-generierten Datenkennzeichnungen, wobei der Trainingsdatensatz Bildinformationen jeweils einer Zelle umfasst, oder (ii) zum Bereitstellen von verborgenen Datenkennzeichnungen auf Basis der Ähnlichkeiten zwischen mindestens zwei Datenpunkten eines Trainingsdatensatzes, wobei der Trainingsdatensatz Bildinformationen jeweils einer Zelle umfasst, und einer daraus abgeleiteten Pseudozeit, sowie von mit dem Trainingsdatensatz assoziierten Experten-generierten Datenkennzeichnungen;
- eine Einheit (i) zum Verknüpfen der verborgenen Datenkennzeichnungen und der Experten-generierten Datenkennzeichnungen, wobei die Verknüpfung ein Verbinden aller verborgenen Datenkennzeichnungen untereinander mittels ungerichteter Kanten und ein Verbinden von verborgenen Datenkennzeichnungen und Experten-generierten Datenkennzeichnungen mittels gerichteter Kanten umfasst, oder (ii) zum Verknüpfen der verborgenen Datenkennzeichnungen und der Experten-generierten Datenkennzeichnungen, wobei die Verknüpfung ein Verbinden aller Datenkennzeichnungen mittels gerichteter Kanten umfasst;
- eine Einheit zum Erhalten von angepassten verborgenen Datenkennzeichnungen auf Basis des probabilistischen graphischen Modells.

**9.** Verwendung eines trainierten probabilistischen graphischen Modells, welches gemäß einem Verfahren nach einem der Ansprüche 1, 3 oder 5 bereitgestellt wurde, zur Überprüfung und/oder Verbesserung der Konsistenz von Datenkennzeichnungen bei der medizinischen Bildverarbeitung zur Zellklassifizierung, wobei vorzugsweise das trainierte probabilistische graphische Modell verwendet wird, um auf Basis einer abgeleiteten verborgenen Datenkennzeichnung eine Experten-generierte Datenkennzeichnung von Bildinformationen zu korrigieren oder zu bestätigen.

**10.** Computer-implementiertes Verfahren zur Überprüfung und/oder Verbesserung der Konsistenz von Datenkennzeichnungen bei der medizinischen Bildverarbeitung zur Zellklassifizierung, umfassend die Schritte:

- Bereitstellen des Bildes einer Zelle;
- Bereitstellen einer vorhandenen Experten-generierten Datenkennzeichnung;
- Errechnen eines Merkmalsraumes für das Bild;
- Anwenden eines Verfahrens auf Basis eines trainierten probabilistischen graphischen Modells auf die errechneten Merkmale des Bildes;
- Verknüpfen einer Datenkennzeichnung mit dem Bild, wobei das Verknüpfen eine Korrektur oder Bestätigung der bereits vorhandenen Experten-generierten Datenkennzeichnung für dieses Bild umfasst;
- Ausgabe und/oder Speicherung des Bildes zusammen mit der korrigierten oder bestätigten verknüpften Datenkennzeichnung.

**11.** Computer-implementiertes Verfahren nach Anspruch 10, wobei das trainierte probabilistische graphische Modell gemäß einem Verfahren nach einem der Ansprüche 1, 3 oder 5 bereitgestellt wurde und/oder das anzuwendende Verfahren ein Verfahren nach einem der Ansprüche 2, 4 oder 6 ist.

12. Computer-implementiertes Verfahren nach Anspruch 10 oder 11, wobei eine Korrektur einer bereits vorhandenen Experten-generierten Datenkennzeichnung zu einer Feedbackanfrage bei einem Experten bezüglich der Datenkennzeichnungsdiskrepanz führt.

13. Datenverarbeitungsvorrichtung die ausgebildet ist, die Konsistenz von Datenkennzeichnungen bei der medizinischen Bildverarbeitung zur Zellklassifizierung zu überprüfen und/oder zu verbessern, wobei die Vorrichtung mindestens einen Prozessor und einen Speicher aufweist, wobei der mindestens eine Prozessor eingerichtet ist, um einen Programmcode aus dem Speicher zu laden und auszuführen und basierend auf dem Ausführen des Programmcodes die folgenden Schritte auszuführen:

    - Bereitstellen des Bildes einer Zelle;
    - Bereitstellen einer vorhandenen Experten-generierten Datenkennzeichnung;
    - Errechnen eines Merkmalsraumes für das Bild;
    - Anwenden eines Verfahrens, vorzugsweise eines Verfahrens nach einem der Ansprüche 2, 4 oder 6, auf Basis eines trainierten probabilistischen graphischen Modells auf die errechneten Merkmale des Bildes, vorzugsweise auf Basis eines trainierten probabilistischen graphischen Modell das gemäß einem Verfahren nach einem der Ansprüche 1, 3 oder 5 bereitgestellt wurde;
    - Verknüpfen einer Datenkennzeichnung mit dem Bild, wobei das Verknüpfen eine Korrektur oder Bestätigung der bereits vorhandenen Experten-generierten Datenkennzeichnung für dieses Bild umfasst;
    - Ausgabe und/oder Speicherung des Bildes zusammen mit der korrigierten oder bestätigten verknüpften Datenkennzeichnung.

14. Computerprogrammprodukt mit einem Computerprogramm, welches direkt in eine Speichereinrichtung eines Computers ladbar ist, mit Programmabschnitten, um alle Schritte eines Verfahrens nach einem der Ansprüche 1 bis 6 oder 10 bis 12 auszuführen, wenn das Computerprogramm in dem Computer ausgeführt wird.

15. Computerlesbares Medium, auf welchem von einem Computer einlesbare und ausführbare Programmabschnitte gespeichert sind, um alle Schritte eines Verfahrens nach einem der Ansprüche 1 bis 6 oder 10 bis 12 auszuführen, wenn die Programmabschnitte von dem Computer ausgeführt werden.

FIG 1

FIG 2

FIG 3

FIG 4

FIG 5

EP 3 719 811 A1

FIG 6

EP 3 719 811 A1

FIG 7

EP 3 719 811 A1

FIG 8

## FIG 9

## FIG 10

## FIG 11

**Europäisches Patentamt**
**European Patent Office**
**Office européen des brevets**

# EUROPÄISCHER RECHERCHENBERICHT

Nummer der Anmeldung

EP 19 16 6679

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | Boqian Wu ET AL: "Multi-Scale Deep Neural Network for Mitosis Detection in Breast Cancer Histological Images", <br><br> , <br> 9. November 2017 (2017-11-09), XP055623715, <br> DOI: 10.20944/preprints201711.0063.v1 <br> Gefunden im Internet: <br> URL:https://www.preprints.org/manuscript/201711.0063/v1 <br> [gefunden am 2019-09-19] <br> * Zusammenfassung * <br> * Kapitel "3.1 Dataset"; <br> Kapitel "2.2 Multi-scale Fused Fully Convolutional Network"; Kapitel "2.3 FF-CNN+CRF model" * <br> ----- | 1-15 | INV. <br> G16H50/70 <br> G16H30/40 |
| A | YIHUA CHEN ET AL: "Similarity-based Classification: Concepts and Algorithms", JOURNAL OF MACHINE LEARNING RESEARCH, MIT PRESS, CAMBRIDGE, MA, US, <br> Bd. 10, 31. März 2009 (2009-03-31), Seiten 747-776, XP058264234, <br> ISSN: 1532-4435 <br> * das ganze Dokument, inbesondere Abstract und Kapitel "I. Introduction" * <br> ----- <br><br> -/-- | 1-15 | |

| | | RECHERCHIERTE SACHGEBIETE (IPC) |
|---|---|---|
| | | G16H |

Der vorliegende Recherchenbericht wurde für alle Patentansprüche erstellt

| Recherchenort | Abschlußdatum der Recherche | Prüfer |
|---|---|---|
| München | 20. September 2019 | Heidrich, Alexander |

EPO FORM 1503 03.82 (P04C03)

Seite 1 von 4

**Europäisches Patentamt**
**European Patent Office**
**Office européen des brevets**

# EUROPÄISCHER RECHERCHENBERICHT

Nummer der Anmeldung

EP 19 16 6679

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | FILIPPO PICCININI ET AL: "Advanced Cell Classifier: User-Friendly Machine-Learning-Based Software for Discovering Phenotypes in High-Content Imaging Data", CELL SYSTEMS, Bd. 4, Nr. 6, 28. Juni 2017 (2017-06-28), Seiten 651-655.e5, XP055624626, US ISSN: 2405-4712, DOI: 10.1016/j.cels.2017.05.012 * das ganze Dokument, insbesondere Seite 1, "Highlights" * ----- | 1-15 | |
| A | AN-AN LIU ET AL: "A Semi-Markov Model for Mitosis Segmentation in Time-Lapse Phase Contrast Microscopy Image Sequences of Stem Cell Populations", IEEE TRANSACTIONS ON MEDICAL IMAGING, IEEE SERVICE CENTER, PISCATAWAY, NJ, US, Bd. 31, Nr. 2, 26. September 2011 (2011-09-26), Seiten 359-369, XP011491026, ISSN: 0278-0062, DOI: 10.1109/TMI.2011.2169495 * das ganze Dokument, insbesondere Abstract; S. 360, Abschnitt "B. Our Approach"; Fig. 1 * ----- -/-- | 1-15 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 20. September 2019 | Heidrich, Alexander |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Seite 2 von 4

Europäisches
Patentamt

European
Patent Office

Office européen
des brevets

**EUROPÄISCHER RECHERCHENBERICHT**

Nummer der Anmeldung

EP 19 16 6679

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | NIE WEIZHI ET AL: "Modeling Temporal Information of Mitotic for Mitotic Event Detection", IEEE TRANSACTIONS ON BIG DATA, IEEE, Bd. 3, Nr. 4, 19. Juli 2017 (2017-07-19), Seiten 458-469, XP011673467, DOI: 10.1109/TBDATA.2017.2723395 [gefunden am 2017-11-28] * das ganze Dokument, insbesondere Abstract, Fig. 2 * ----- | 1-15 | |
| A | US 2011/243417 A1 (MADABHUSHI ANANT [US] ET AL) 6. Oktober 2011 (2011-10-06) * Anspruch 1 * ----- | 1-15 | |
| A | LIANG-CHIEH CHEN ET AL: "DeepLab: Semantic Image Segmentation with Deep Convolutional Nets, Atrous Convolution, and Fully Connected CRFs", ARXIV.ORG, CORNELL UNIVERSITY LIBRARY, 201 OLIN LIBRARY CORNELL UNIVERSITY ITHACA, NY 14853, 2. Juni 2016 (2016-06-02), XP080705599, * das ganze Dokument, insbesondere Abstract und Fig. 1 * ----- | 1-15 | |
| A | LIU YIMING ET AL: "Automatic Segmentation of Cervical Nuclei Based on Deep Learning and a Conditional Random Field", IEEE ACCESS, Bd. 6, 19. September 2018 (2018-09-19), Seiten 53709-53721, XP011692996, DOI: 10.1109/ACCESS.2018.2871153 [gefunden am 2018-10-15] * das ganze Dokument, insbesondere Abstract und Fig. 1 * ----- | 1-15 | RECHERCHIERTE SACHGEBIETE (IPC) |

-/--

Der vorliegende Recherchenbericht wurde für alle Patentansprüche erstellt

| Recherchenort | Abschlußdatum der Recherche | Prüfer |
|---|---|---|
| München | 20. September 2019 | Heidrich, Alexander |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
...............................................................................
& : Mitglied der gleichen Patentfamilie, übereinstimmendes
    Dokument

EPO FORM 1503 03.82 (P04C03)

Europäisches
Patentamt

European
Patent Office

Office européen
des brevets

**EUROPÄISCHER RECHERCHENBERICHT**

Nummer der Anmeldung

EP 19 16 6679

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | ALBAYRAK ABDULKADIR ET AL: "Mitosis detection using convolutional neural network based features", 2016 IEEE 17TH INTERNATIONAL SYMPOSIUM ON COMPUTATIONAL INTELLIGENCE AND INFORMATICS (CINTI), IEEE, 17. November 2016 (2016-11-17), Seiten 335-340, XP033060964, DOI: 10.1109/CINTI.2016.7846429 [gefunden am 2017-02-07] * das ganze Dokument insbesondere Abstract und Fig. 3 * ----- | 1-15 | |
| | | | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 20. September 2019 | Heidrich, Alexander |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

Seite 4 von 4

## ANHANG ZUM EUROPÄISCHEN RECHERCHENBERICHT
## ÜBER DIE EUROPÄISCHE PATENTANMELDUNG NR.

EP 19 16 6679

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-09-2019

| Im Recherchenbericht angeführtes Patentdokument | Datum der Veröffentlichung | Mitglied(er) der Patentfamilie | Datum der Veröffentlichung |
|---|---|---|---|
| US 2011243417 A1 | 06-10-2011 | US 2011243417 A1<br>WO 2010027476 A1 | 06-10-2011<br>11-03-2010 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **VAN DER MEER et al.** *J Clin Pathol,* 2007, vol. 60 (7), 838-839
- **REID ; WERNISCH.** *Bioinforamtics,* 2016, vol. 32 (19), 2973-2980
- **KONDO et al.** *Proceedings of the Eighth Workshop on Statistical Machine Translation,* 2013, 503-511